# EUROPÄISCHE PATENTSCHRIFT

(11) **EP 0 868 421 B1**
(45) Veröffentlichungstag und Bekanntmachung des Hinweises auf die Patenterteilung: **02.08.2006**
(21) Anmeldenummer: 96945721.7
(22) Anmeldetag: 13.11.1996
(51) Int. Cl.: C07D 233/32

(54) **VERFAHREN ZUR HERSTELLUNG VON (METH)ACRYLSÄUREESTERN**
PROCESS FOR THE PREPARATION OF (METH)ACRYLIC ACID ESTERS
PROCEDE DE PREPARATION D'ESTERS D'ACIDE (METH)ACRYLIQUE

(30) Priorität: 16.12.1995 DE 19547099
(43) Veröffentlichungstag der Anmeldung: 07.10.1998
(73) Patentinhaber: Röhm GmbH & Co. KG, 64293 Darmstadt (DE)
(72) Erfinder: KNEBEL, Joachim, D-64293 Darmstadt (DE); MERBACH, Ralf, D-64572 Büttelborn (DE)
(86) Internationale Anmeldenummer: PCT/DE1996/002161
(87) Internationale Veröffentlichungsnummer: WO 1997/022592

(56) Entgegenhaltungen:
- EP-A- 0 571 851
- EP-A- 0 619 309
- EP-A- 0 650 962
- CHEMICAL ABSTRACTS, vol. 84, no. 20, 17.Mai 1976 Columbus, Ohio, US; abstract no. 136271f, Y. HAMAMOTO ET AL.: "(Dimethylamino)ethyl methacrylate or acrylate." Seite 17; Spalte 2; XP002031284 & JP 75 142 513 A (KYOWA GAS CHEMICAL INDUSTRY CO., LTD.) 17.November 1975

## Beschreibung

### Gebiet der Erfindung

Die vorliegende Erfindung betrifft ein neues und verbessertes Verfahren zur Herstellung von Acryl- oder Methacrylsäureestern der Formel 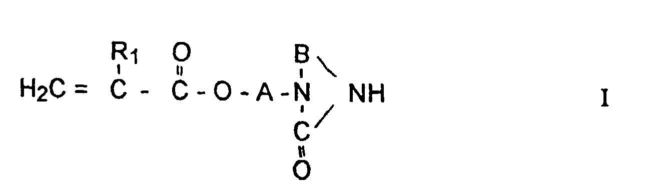 in der R₁ für Wasserstoff oder eine Methylgruppe steht und A und B unverzweigte oder verzweigte Alkylengruppen mit 2 bis 5 C-Atomen bedeuten.

### Stand der Technik

Verbindungen der Formel I lassen sich nach dem in der US-Patentschrift 2 871 223 beschriebenen Verfahren durch Umsetzung von Acryl- oder Methacrylsäurechlorid mit Hydroxialkylimidazolidin-2-onen in Gegenwart tertiärer Stickstoffbasen, wobei stöchiometrische Mengen der Hydrochloride der tertiären Stickstoffbasen mit anfallen, erhalten.

In EP 0619309 und EP 0650962 werden Verfahren zur Herstellung von Alkylimidazolidon (Meth)acrylaten beschrieben. Die Umsetzungen erfolgen in EP 0619309 in Gegenwart von Katalysatoren, die unter den Chelaten von Calcium mit 1,3-Dicarbonylverbindungen ausgewählt sind. In EP 0650962 wird mit einem Magnesiumalkoholat katalysiert.

Hamamoto et al. (Chemical Abstracts, vol.84, no. 20, 17. Mai 1976) beschreiben die Synthese von 2-(Dimethylamino)ethylacrylat bzw. - methacrylat in Gegenwart von Ca(OH)₂ oder CaO.

Bei dem aus der EP 0 236 994 A1 bekannten Verfahren zur Herstellung von Acryl- und Methacrylestern der Formel I werden Acryl- oder Methacrylsäureester mit 1-(Hydroxialkyl)-imidazolidin-2-onen in Gegenwart von Titanalkoholaten oder Chelatverbindungen der Metalle Titan, Zirkonium, Eisen und Zink mit 1,3-Dicarbonylverbindungen als Umesterungskatalysatoren umgesetzt.

In den EP-A 0 433 135 und EP-A 0 453 638 werden für die Umesterung von Acryl- und Methacrylestern mit Hydroxialkylimidazolidin-2-onen zu Verbindungen der Formel I Diorganozinnoxid-Verbindungen als Umesterungs-Katalysatoren beansprucht.

In der Regel muß aus den Ansätzen nach Beendigung der Umsetzung der Metallkatalysator abgetrennt werden. Dies geschieht vorteilhaft, so z.B. bei Verwendung von Tetraalkyltitanaten oder von Dialkylzinnoxiden, durch Zusetzen von Wasser. Aus den Titanaten entstehen dabei Metall(hydr)oxide, wie z.B. TiO₂, die beispielsweise durch Filtrieren oder Zentrifugieren abgetrennt werden. Diese hydrolysierten Umesterungs-katalysatoren können nach Abtrennung nicht mehr als solche erneut eingesetzt werden.
Die Dialkylzinnoxide lassen sich durch den Wasserzusatz zwar als solche wieder abtrennen und als Umesterungskatalysatoren erneut einsetzen. Jedoch muß dazu eine relativ große Wassermenge zunächst eingebracht werden, die dann wieder aus dem Reaktionsprodukt entfernt werden muß. Die Umsetzung kann nach der deutschen Patentanmeldung P 42 17 124.5 auch in Gegenwart von Gemischen von Alkali-/Erdalkalimetallverbindungen, die im wesentlichen als Oxide, Hydroxide, Carbonate und/oder als Salze von Carbonsäuren eingesetzt werden, durchgeführt werden. Die als Katalysatoren vorhandenen Alkali-/ErdalkaliVerbindungen sind ohne Zusetzen von Wasser abtrennbar. Die Menge an katalytisch aktiven Verbindungsgemischen beträgt 0,01 - 10 Gew.-%, bezogen auf das Reaktionsgemisch.

Trotz der vorteilhaft hohen Reaktionsgeschwindigkeit, die mit Alkali-Erdalkali-Katalysatoren erreicht wird, stagnieren diese Systeme nach einem ca. 80 %igen Hydroxialkylimidazolidin-2-on-Umsatz, so daß der Restalkoholgehalt im Umsetzungsgemisch relativ hoch ist. Auch die Bildung von N-(Methacryloyloxiethyl)-N'-(methacryloyl)ethylenharnstoff einer bifunktionellen Methacryl- und damit bei Polymerisationen vemetzend wirkenden Verbindung, ist mit ca. 10 % der Umesterungsverbindungen sehr hoch und ist zu niedrigeren Anteilen verbesserungsbedürftig.

DE-OS 3013927 (BASF) beschreibt eine polymeranaloge Reaktion mit ungefähr 100 000 ppm Calciumhydroxid als Katalysator.

DE 2238208 beschreibt die Umesterung von bakteriziden Chinoxalinderivaten unter Calciumhydroxid- oder Bariumhydroxidkatalyse.

### Aufgabe und Lösung

Der Erfindung lag die Aufgabe zugrunde, ein katalytisches Verfahren zur Herstellung von Acryl- oder Methacrylsäureestern der Formel I durch Alkoholyse von (Meth)acrylsäurealkylestern mit Hydroxialkylimidazolidin-2-onen zu finden, das mit guter Reaktionsgeschwindigkeit auch im Bereich der Endumesterung abläuft, und bei dem der verwendete Katalysator ohne die Zugabe von Wasser aus dem Reaktionsgemisch abgetrennt und gegebenenfalls als solcher wieder eingesetzt werden kann.

Es wurde nun gefunden, daß die Umsetzung überraschenderweise vorteilhaft mit Calciumhydroxid in einer Menge von weniger als 250 ppm, bezogen auf die Gesamtmenge des Reaktionsansatzes durchgeführt werden kann.

Die Erfindung betrifft ein Verfahren zur Herstellung von (Meth)acrylester der Formel I 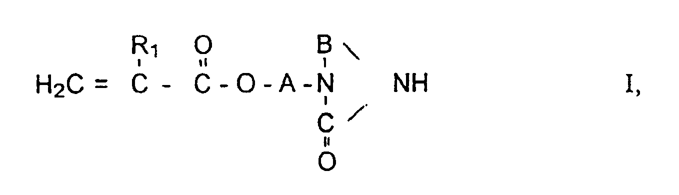 in der R₁ für Wasserstoff oder eine Methylgruppe steht und A und B unverzweigte oder verzweigte Alkylengruppen mit 2 bis 5 C-Atomen bedeuten, durch Umsetzung eines Acrylsäureesters oder Methacrylsäureesters der Formel II 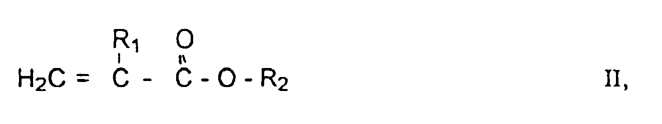 in der R₁ die oben angegebene Bedeutung und R₂ die Bedeutung eines Alkylrestes mit 1 bis 4 C-Atomen haben, mit einer heterocyclischen Verbindung der Formel III 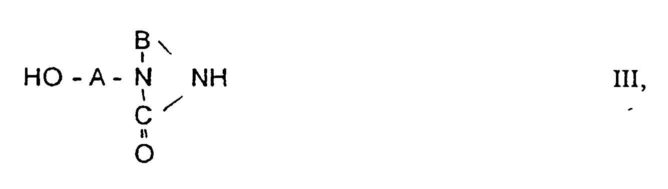 worin A und B die oben angegebene Bedeutung haben das dadurch gekennzeichnet ist, daß die Umsetzung eines Esters entsprechend der Formel II mit einer heterocyclischen Verbindung der Formel III zu einem Acryl-oder Methacrylester der Formel I in Gegenwart eines Katalysators, der Calciumhydroxid enthält, durchgeführt wird.

Ein besonderer Vorteil des neuen Verfahrens ist, daß hohe Umsätze erreicht werden, und daß ohne Zugabe von Wasser (wir setzen Tonsil zu) das calciumhaltige Katalysatorsystem, das weitgehend quantitativ in dem Reaktionsgemisch aufgeschlämmt ist, z.B. durch Filtration abgetrennt werden kann. Als Hilfsmittel zur Abtrennung von gelösten Katalysatoranteilen wird Tonsil® eingesetzt. (Lieferant: Südchemie AG)

Verbindungen der Formel I sind wertvolle Comonomere und werden beispielsweise bei der Herstellung von Polymerdispersionen aus Vinylmonomeren eingesetzt, die vor allem als Bindemittel z.B. in Lacken, oder als Lederhilfsmittel Verwendung finden. Comonomere der Formel I verleihen Copolymerisaten eine gewünschte Hydrophilie und können in wärmehärtbaren Harzen mit ihrer Imidgruppe als Formaldehydfänger fungieren.

Der Erfolg des erfindungsgemäßen Verfahrens ist überraschend, da aufgrund der Bifunktionalitäten von I und III bei deren Umsetzung in weiteren Reaktionen, wie Additionsreaktionen analog einer Michaeladdition an die Doppelbindung, oder in einer Amidbildung durch Reaktion des Acryl- oder Methacrylesters der Formel I mit der NH-Gruppierung einer Verbindung der Formel III in Gegenwart des Katalysators zu rechnen war. Die erfindungsgemäße Umsetzung von Acryl- und Methacrylestern der Formel II mit den Alkoholen der Formel III verläuft sehr selektiv zu Verbindungen der Formel I.

Nach dem erfindungsgemäßen Verfahren werden Verfahrensprodukte der Formel I erhalten, die ohne preislich und qualitativ belastende Abtrennverfahren, unmittelbar z.B. als Lösung in dem Acryl- bzw. Methacrylester II, für die Verwendung als Comonomere, vor allem bei der Herstellung von Dispersionspolymerisaten, eingesetzt werden können. Die Verbindungen I können nach dem vorliegenden Verfahren auch als Festprodukte hergestellt werden, z. B. durch Einengen aus der Lösung.

### Durchführung der Erfindung

Für die Herstellung der Verbindungen I nach dem erfindungsgemäßen Verfahren werden Acryl- oder Methacrylsäureester der Formel II verwendet, in denen R₂ ein Alkylrest mit 1 bis 4 Kohlen-stoffatomen bedeutet. Beispielsweise seien dazu Propylacrylat, n-Butylacrylat, Ethylmethacrylat, i-Propylmethacrylat, i-Butylmethacrylat, n-Butylmethacrylat und vor allem Methylmethacrylat genannt.

Als Ausgangsstoffe der Formel III kommen solche Verbindungen in Frage, in denen A oder B eine verzweigte oder unverzweigte Alkylengruppe mit 2 bis 5 Kohlenstoffatomen darstellt, z.B. -(CH₂)₂-, -(CH₂)₃-, -(CH₂)₄-, -CH₂CH(CH₃)CH₂-, -CH₂C(CH₃)₂CH₂-. Die Ringgliederzahl des Heterocyclus beträgt vorzugsweise 5 und 6. Besonders vorteilhaft wird als Verbindung III 1-(2-Hydroxiethyl)-imidazolidin-2-on, das z. B. nach der US-PS 3 254 075 gut und technisch aus Aminoethylethanolamin und Harnstoff herstellbar ist, verwendet.

Als Calciumverbindungen, die dem Reaktionssystem als Katalysatoren bzw. katalysatorbildende Vorläufer zugesetzt werden, seien die zweiwertigen Calciumverbindungen, wie beispielsweise Calciumhydroxid, Calciumalkoholate, genannt.

Die den Katalysator bzw. das Katalysatorsystem bildenden Calciumverbindungen werden zweckmäßig in katalytischen Mengen, im allgemeinen von nicht mehr als 250 ppm bezogen auf die Summe der Reaktionspartner II und III angewendet. Eine hohe Selektivität an Produkt I mit R₁ = CH₃, A und B = -(CH₂)₂- wird z.B. mit 250 ppm Ca(OH)₂ bezogen auf die Gesamtansatzmenge bei der Umesterung von Methylmethacrylat mit der dazu entsprechenden Verbindung III erreicht.

Vorteilhafterweise geschieht die Verwendung der Katalysatoren in feiner Verteilung z. B. in pulver- bzw. in feinkristalliner Form.
Die Umsetzung von Acrylestern und/oder Methacrylestern der Formel II mit den Alkoholen der Formel III (Alkoholyse) wird bei Temperaturen zwischen 30 und 180 Grad C, vor allem zwischen 50 und 130 Grad C, in Gegenwart von nicht mehr als 250 ppm der Calciumverbindung, berechnet auf das Gewicht des Reaktionsgemisches, durchgeführt.

Formal reagieren äquimolare Mengen der Reaktionspartner II und III zu den gewünschten Endprodukten I. In der Praxis hat es sich jedoch als zweckmäßig erwiesen, während der Umsetzung die Ausgangsester II stets im Überschuß zu halten. Sie werden in Mengen von 1 bis 20, vorzugsweise 2 bis 10, insbesondere 3 bis 6 Mol pro Mol III eingesetzt.

Zur Vermeidung von Polymerisationsverlusten ist es zweckmäßig, die Umsetzung und Aufarbeitung des Reaktionsgemisches in Gegenwart von Polymerisationsinhibitoren wie z.B. Phenothiazin, Hydrochinonmonomethylether und insbesondere Sauerstoff, durchzuführen.

Die Umsetzung kann unter Normaldruck, Unter- oder Überdruck ablaufen. Sie kann diskontinuierlich oder kontinuierlich erfolgen. Die Ausgangsstoffe II und III werden beispielsweise gemeinsam in Gegenwart von Calciumverbindungen zum Sieden erhitzt und dabei kontinuierlich der abgespaltene Alkohol R₂OH, gegebenenfalls in Form seines Azeotrops mit dem Ester II abdestilliert. Je nach Reaktionstemperatur, Katalysator bzw. Katalysatormenge liegen die Umsetzungszeiten im Bereich von ca. 2 bis 10 Stunden. Es ist auch möglich, die Umsetzung in Gegenwart eines inerten Lösungsmittels, z.B. Toluol oder Cyclohexan, durchzuführen, was sich aber normalerweise erübrigt.

Nach Beendigung der Umsetzung kann überschüssiger monomerer Ester II vollkommen oder teilweise durch Abdestillieren entfernt werden.
Der dispergierte Katalysator wird üblicherweise durch Filtration, vorteilhaft vor dem Abdestillieren des meist überschüssigen monomeren Esters II, entfernt. Die Abtrennung kann aber auch erst nach teilweiser oder vollständiger Entfernung von überschüssigem monomerem Ester II erfolgen. Der in abfiltrierter Form zurückgewonnene Katalysator kann dann, gegebenenfalls nach Trocknung, wieder in weiteren Alkoholyseansätzen eingesetzt werden.

Ein bevorzugtes Umsetzungsprodukt ist ein solches, das aus Methylmethacrylat und 1-(2-Hydroxiethyl)-imidazolidin-2-on (Hydroxyethylethylenharnstoff) entsteht und so der Formel I mit R₁ = CH₃, A = -(CH2)2- und B = -(CH₂)₂-entspricht.

### BEISPIELE

### Beispiel 1

In einen 2 I-Rundkolben mit mechanischer Rührung, Lufteinleitung, Sumpftemperaturanzeige und aufgesetzter Füllkörperkolonne (⌀: 35 mm, Höhe 55 cm, 8 x 8 mm - Raschigringe) sowie automatischen Kolonnenkopf mit Rückfluß- und Destillatkühler gibt man 1100 g (11 mol) Methylmethacrylat, 286 g (2,2 mol) Hydroxyethylethylenharnstoff und als Inhibitoren 0,35 g Hydrochinonmonomethylether sowie 0,09 g Phenothiazin.
Man erhitzt zum Sieden und destilliert über die Kolonne zunächst ein Methylmethacrylat-Wasser-Azeotrop ab, bis die Kopf-temperatur 99 °C erreicht. Der Ansatz wird um ca. 10 °C abgekühlt, 0,35 g Calciumhydroxid und die dem Azeotropdestillat äquivalente Masse Methylmethacrylat zugegeben.
Erneut wird zum Sieden erhitzt und das entstehende Methylmethacrylat-Methanolazeotrop bei einem Rücklauf-verhältnis von 2 : 1 bis zu einer maximalen Kopftemperatur von 70 °C, später bei einem Rücklaufverhältnis von 10 : 1 bis zum Erreichen einer konstanten Kopftemperatur (99 °C) abdestilliert. Die Reaktion ist nach 6 h beendet. Der Ansatz wird auf 80 °C abgekühlt und durch Zugabe von Methylmethacrylat bis zu einer Gesamtmasse von 1742 g auf eine 25 %ige Lösung des Produkts in Methylmethacrylat eingestellt. Man gibt 3,5 g Tonsil L80FF® (Südchemie) hinzu und rührt 15 min nach. Anschließend kühlt man auf Raumtemperatur ab und klärt den Ansatz durch Druckfiltration (Seitz Druckfilter Ø = 14 cm; Filterschicht T 1000 (Seitz), p < 0,4 bar). Das Filtrat hat nach gaschromatographischer Analyse die folgende Zusammensetzung:

| | |
|---|---|
| Methylmethacrylat: | 72,5 % |
| Hydroxyethylethylenharnstoff: | 1,4 % |
| Methacryloyloxyethylethylenharnstoff: | 23,7 % |
| N-(Methacryloyloxyethyl)-N'-(methacryloyl)ethylenharnstoff:1,2 | % |

### Beispiel 2

Durchführung wie Beispiel 1, aber unter Weglassen des Entwässerungsschritts. Reaktionszeit: 5,3 h. Das Produkt ist nach gaschromatographischer Analyse wie folgt zusammengesetzt:

| | |
|---|---|
| Methylmethacrylat: | 71,8 % |
| Hydroxyethylethylenharnstoff: | 1,7 % |
| Methacryloyloxyethylethylenharnstoff: | 24,0 % |
| N-(Methacryloyloxyethyl)-N'-(methacryloyl)ethylenharnstoff: | 1,2 % |
| Platin-Cobalt-Farbzahl: | 22 |
| Säurezahl: | 0,05 |

### Beispiel 3

Durchführung wie Beispiel 2, aber unter Verwendung von 0,55 g Calciumhydroxid. Reaktionszeit: 5,5 h. Das Produkt hat nach gaschromatographischer Analyse die folgende Zusammensetzung:

| | |
|---|---|
| Methylmethacrylat: | 70,5 % |
| Hydroxyethylethylenharnstoff: | 1,0 % |
| Methacryloyloxyethylethylenharnstoff: | 24,4 % |
| N-(Methacryloyloxyethyl)-N'-(methacryloyl)ethylenhamstoff: | 2,0 % |

### Beispiel 4:

Durchführung wie in Beispiel 2, aber unter Verwendung von 0,28 g (200 ppm bez. auf Gesamteinwaage) Calciumhydroxid. Reaktionszeit: 6,0 h. Das Produkt hat nach gaschromatographischer Analyse die folgende Zusammensetzung:

| | |
|---|---|
| Methylmethacrylat: | 71,3 % |
| Hydroxethylethylenharnstoff: | 1,6 % |
| Methacryloyloxyethylethylenharnstoff: | 25,1 % |
| N-(Methacryloyloxyethyl)-N'-(methacryloyl)ethylenharnstoff: | 0,7 % |

## Patentansprüche

1. Verfahren zur Herstellung von (Meth)acrylestern der Formel I 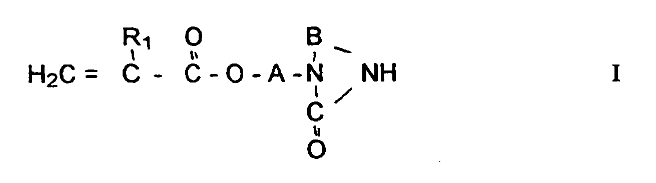 in der R₁ für Wasserstoff oder eine Methylgruppe steht und A und B unverzweigte oder verzweigte Alkylengruppen mit 2 bis 5 C-Atomen bedeuten, durch Umsetzung eines Acrylsäureesters oder Methacrylsäureesters der Formel II 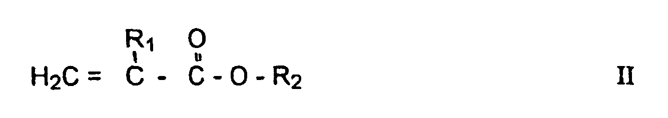 in der R₁ oben angegebenen Bedeutung und R₂ die Bedeutung eines Alkylrestes mit 1 bis 4 C-Atomen haben, mit einer heterocyclischen Verbindung der Formel III 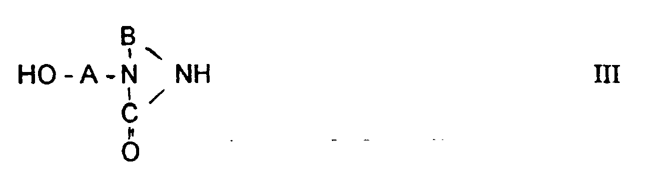 worin A und B die angegebenen Bedeutungen haben, **dadurch gekennzeichnet, daß** die Umesterung von II mit III zu einem Acryl- oder Methacrylester der Formel I in Gegenwart eines Katalysators, der Calciumhydroxid enthält, durchgeführt wird.

2. Verfahren nach Anspruch 1, **dadurch gekennzeichnet, daß** die Menge des Katalysatorsystems 1 ppm bis 250 ppm, bezogen auf das Reaktionsgemisch, beträgt.

3. Verfahren nach dem Anspruch 2, **dadurch gekennzeichnet, daß** die Menge des Katalysatorsystems 10 bis 150 ppm, bezogen auf das Reaktionsgemisch, beträgt.

4. Verfahren nach den Ansprüchen 1 bis 3, **dadurch gekennzeichnet, daß** die Umsetzung von Acryl- oder Methacrylestern der Formel II mit 1-(2-Hydroxiethyl)-imidazolidin-2-on (2-(Hydroxyethyl)ethylenharnstoff) durchgeführt wird.

5. Verfahren nach den Ansprüchen 1 bis 4, **dadurch gekennzeichnet, daß** Methylmethacrylat als Verbindung der Formel II mit einem Alkohol der Formel III umgesetzt wird.

## Claims

1. Process for the preparation of (meth) acrylic esters of the formula I 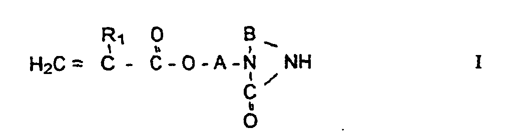 in which R₁ represents hydrogen or a methyl group and A and B denote straight-chain or branched alkylene groups having 2 to 5 C atoms, by reacting an acrylic ester or methacrylic ester of the formula II 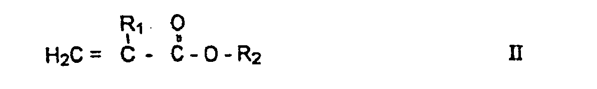 in which R₁ has the above-mentioned meaning and R₂ denotes an alkyl radical having 1 to 4 C atoms, with a heterocyclic compound of the formula III 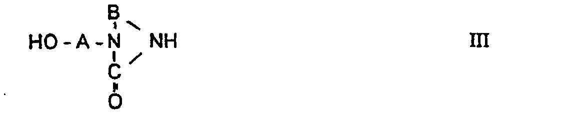 in which A and B have the stated meanings, **characterized in that** the transesterification of II with III to give an acrylic or methacrylic ester of the formula I is carried out in the presence of a catalyst which contains calcium hydroxide.

2. Process according to Claim 1, **characterized in that** the amount of the catalyst system is from 1 ppm to 250 ppm, based on the reaction mixture.

3. Process according to Claim 2, **characterized in that** the amount of the catalyst system is from 10 to 150 ppm, based on the reaction mixture.

4. Process according to Claims 1 to 3, **characterized in that** the reaction of acrylic or methacrylic esters of the formula II with 1-(2-hydroxyethyl)imidazolidin-2-one (2-(hydroxyethyl)-ethyleneurea) is carried out.

5. Process according to Claims 1 to 4, **characterized in that** methyl methacrylate, as a compound of the formula II, is reacted with an alcohol of the formula III.

## Revendications

1. Procédé de préparation d'esters (méth)acryliques de formule I 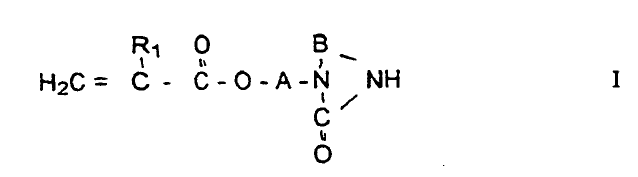 dans laquelle R₁ représente un atome d'hydrogène ou un groupe méthyle et A et B représentent des groupes alkylènes non ramifiés ou ramifiés renfermant de 2 à 5 atomes de carbone, par réaction d'un ester d'acide acrylique ou d'un ester d'acide méthacrylique de formule II 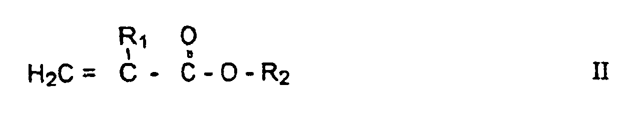 dans laquelle R₁ présente la signification indiquée ci-dessus et R₂ représente un radical alkyle renfermant de 1 à 4 atomes de carbone, avec un composé hétérocyclique de formule III 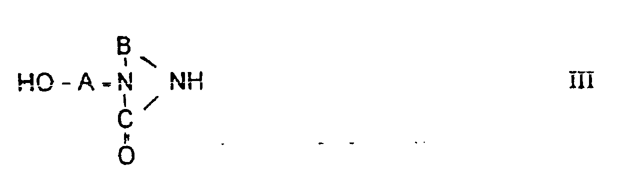 dans laquelle A et B présentent les significations indiquées, **caractérisé en ce que** l'estérification de II avec III pour donner lieu à un ester acrylique ou méthacrylique de formule I est réalisée en présence d'un catalyseur, qui contient de l'hydroxyde de calcium.

2. Procédé selon la revendication 1, **caractérisé en ce que** la quantité du système de catalyseur est de 1 ppm à 250 ppm, par rapport au mélange réactionnel.

3. Procédé selon la revendication 2, **caractérisé en ce que** la quantité du système de catalyseur est de 10 à 150 ppm, par rapport au mélange réactionnel.

4. Procédé selon les revendications 1 à 3, **caractérisé en ce que** la réaction d'esters acryliques ou méthacryliques de formule II est réalisé avec de la 1-(2-hydroxyéthyl)imidazolidin-2-one (2-(hydroxyéthyl)éthylène-urée).

5. Procédé selon les revendications 1 à 4, **caractérisé en ce que**, en tant que composé de formule II, le méthacrylate de méthyle est mis à réagir avec un alcool de formule III.
